# FASCICULE DE BREVET EUROPEEN

(11) **EP 4 405 666 B1**
(45) Date de publication et mention de la délivrance du brevet: **14.10.2026**
(21) Numéro de dépôt: 22792855.3
(22) Date de dépôt: 23.09.2022
(51) Int. Cl.: G01N 21/65, C12N 1/16, G01N 21/3577, G01N 21/3581, G01N 33/02

(54) **MÉTHODE POUR DÉTERMINER UNE QUALITÉ D'UNE MÉLASSE UTILISÉE DANS LA PRODUCTION D'UNE LEVURE**
VERFAHREN ZUR BESTIMMUNG EINER QUALITÄT VON MELASSE, DIE ZUR HERSTELLUNG VON HEFE VERWENDET WIRD
METHOD FOR DETERMINING A QUALITY OF MOLASSES USED IN YEAST PRODUCTION

(30) Priorité: 24.09.2021 FR 2110103
(43) Date de publication de la demande: 31.07.2024
(73) Titulaire: Lesaffre et Compagnie, 75001 Paris (FR)
(72) Inventeur: ALLAIRE, Melvyn, 77144 MONTEVRAIN (FR); CLEMENT-ZIZA, Mathieu, 59130 LAMBERSART (FR); DELINE, Ludovic, 59290 WASQUEHAL (FR)
(74) Mandataire: Plasseraud IP
(86) Numéro de dépôt international: PCT/FR2022/051797
(87) Numéro de publication internationale: WO 2023/047066

(56) Documents cités:
- DE-A1- 102019 107 629
- CHAPANYA PISITTINEE ET AL: "Temperature Compensation on Sugar Content Prediction of Molasses by Near-Infrared Spectroscopy (NIR)", SUGAR TECH, SPRINGER INDIA, NEW DELHI, vol. 21, no. 1, 3 July 2018 (2018-07-03), pages 162 - 169, XP036687381, ISSN: 0972-1525, [retrieved on 20180703], DOI: 10.1007/S12355-018-0635-X
- WANG WEI ET AL: "Qualitative Prediction of Yeast Growth Process Based on Near Infrared Spectroscopy", CHINESE JOURNAL OF ANALYTICAL CHEMISTRY, vol. 45, no. 8, 1 August 2017 (2017-08-01), pages 1137 - 1141, XP085177648, ISSN: 1872-2040, DOI: 10.1016/S1872-2040(17)61030-2

## Description

### Domaine technique

La présente divulgation relève du domaine de la production de levure, et plus particulièrement, elle concerne une méthode pour déterminer une qualité d'une mélasse utilisée comme source de nutriments dans la production d'une levure, cette qualité de mélasse étant relative à une ou plusieurs performances de la levure produite.

Le terme « levure », au singulier ou au pluriel, est un terme générique désignant des microorganismes eucaryotes aptes à provoquer une fermentation de matières organiques. Les levures sont couramment utilisées, que ce soit chez les particuliers ou dans l'industrie agroalimentaire ou pharmaceutique. Par exemple, elles peuvent être employées dans la fabrication du vin, de la bière, ou encore de pâtes levées (e.g., pains). Parmi les levures, on citera de façon non exhaustive les genres *Saccharomyces, Candida, Pichia, Kluyveromyces.* L'expression « souche de levure » désigne une population relativement homogène de cellules de levure. Une souche de levure est obtenue à partir de l'isolement d'un clone. Un clone donne naissance à une population de cellules obtenue à partir d'une seule cellule de levure.

Une levure est généralement produite à partir de la multiplication d'une souche cultivée dans un milieu comprenant un ensemble de matières premières. En milieu industriel, à partir d'une quantité de départ donnée de levure, il est possible d'en produire plusieurs tonnes en sortie. Les matières premières généralement utilisées pour alimenter une production de levure sont un ensemble de nutriments et une source de sucre(s), qui peut être un sirop de sucre, de la mélasse, un mélange de sirops de sucre et/ou de mélasses.

La mélasse est une matière première issue des industries sucrières et elle peut se distinguer suivant deux types de mélasse, la mélasse de betterave ou la mélasse de canne. Les nutriments peuvent être des sources d'azote, de phosphore, de minéraux, d'oligoéléments (e.g., Fe, Zn, etc.), de vitamines (e.g., B, C, etc.), d'eau, ou encore d'air.

Lors de la production de levure, plusieurs facteurs peuvent impacter le rendement ou la qualité de la levure produite. Ces facteurs peuvent être la qualité de la mélasse utilisée ainsi que les différentes proportions de nutriments apportés à la levure. En fonction d'une qualité de mélasse donnée ou d'autres facteurs ou d'un ensemble de facteurs incluant la qualité de la mélasse, il est possible d'ajuster les schémas de production comme par exemple les quantités respectives de chaque nutriment de manière à optimiser le rendement de production et/ou la qualité de la levure en sortie.

Actuellement, la qualité d'une mélasse n'ayant pas encore servi en production ainsi que la qualité de la levure produite à partir de cette mélasse peuvent être déterminées seulement après soit une première production de levure suivie de protocoles de qualification de la levure produite, soit une série de tests miniaturisés en laboratoire permettant d'estimer grossièrement la qualité et d'écarter les qualités atypiques de mélasse. En effet, la qualité d'une mélasse inconnue ou la compensation d'une mélasse connue mais de mauvaise qualité par ajustement des schémas de production ne peuvent se faire qu'après une qualification de la levure obtenue à partir de cette qualité de mélasse.

En sachant qu'une première production peut correspondre à plusieurs centaines de tonnes de levure produites et peut avoir nécessité la mise en fonctionnement de plusieurs sites de production, l'obtention d'un mauvais rendement de production ou d'une levure de mauvaise qualité peut représenter une perte significative pour un industriel.

En parallèle, l'organisation de la production des mélasses en « campagnes sucrières » entraîne le fait que la qualité des mélasses reçues dans le temps est susceptible de variations sensibles, et qu'il n'est pas possible d'anticiper l'impact de ces variations.

Ces variations de qualité et leur impact sur la qualité de la levure sont d'autant moins faciles à anticiper que les mélasses sont stockées dans des cuves pouvant contenir des quantités utilisables pendant plusieurs semaines de production, ce qui rend encore plus difficile la détection d'un lot de mélasse à la qualité atypique qui aurait été transféré dans la cuve de stockage.

Il existe donc un besoin pour déterminer la qualité d'une mélasse avant son utilisation dans une production de levure de manière à éviter la production d'une levure présentant une qualité ou/et des performances non conformes (e.g. à un cahier des charges).

Le document CHAPANYA PISITTINEE ET AL: "Temperature Compensation on Sugar Content Prediction of Molasses by Near-Infrared Spectroscopy (NIR)", SUGAR TECH, SPRINGER INDIA, NEW DELHI, vol. 21, no. 1, 3 juillet 2018 (2018-07-03), pages 162-169, décrit une méthode permettant de déterminer, au moyen de la spectroscopie NIR et de modèles prédictifs, la teneur en sucres fermentescibles de la mélasse afin d'optimiser le rendement en éthanol.

La présente divulgation vient améliorer la situation.

Il est ainsi proposé un procédé, selon la revendication 1, de qualification de mélasse à partir d'une mesure optique, le procédé pouvant comprendre :
- à partir de spectres optiques de référence d'échantillons de mélasses distinctes, associées à des qualités de mélasse respectives, connues, construire par apprentissage automatique un modèle statistique de qualités de mélasse en fonction d'au moins une caractéristique spectrale des spectres de référence, et
- pour un spectre optique courant d'un échantillon d'une mélasse courante, à partir du modèle statistique, identifier ladite au moins une caractéristique spectrale du spectre optique courant et déterminer une qualité de la mélasse courante, ladite qualité de mélasse courante étant relative à au moins une performance de levure obtenue lorsque ladite levure est alimentée avec ladite mélasse courante, dans lequel chaque qualité de mélasse associée respectivement à un échantillon de mélasse distincte est relative à au moins une performance de levure de référence obtenue lorsque ladite levure de référence est alimentée avec ledit échantillon de mélasse distincte.

De manière avantageuse, il est ainsi possible de déterminer la qualité d'une mélasse inconnue à partir de son spectre optique, et d'en déduire au moins une performance de levure de la levure produite à partir de cette mélasse inconnue pour des conditions de production (e.g. température et pression dans le fermenteur) et une souche de levure déterminées.

Ainsi, avantageusement, une mélasse ne présentant pas la qualité requise pour permettre l'obtention d'une levure ayant une ou plusieurs performances cibles peut être écartée avant toute utilisation en production. Selon un autre avantage, à partir de la qualité de mélasse déterminée, il est possible d'adapter les schémas de production comme par exemple en en ajustant les proportions de nutriments nécessaires afin de corriger une mélasse de mauvaise qualité de manière à permettre la ou les performances cibles. En outre, en fonction du degré de mauvaise qualité d'une mélasse, il peut être possible de corriger cette qualité en mélangeant la mélasse avec des mélasses de qualités différentes.

Dans un ou plusieurs modes de réalisations, ladite au moins une performance relative à une qualité de mélasse associée respectivement à un échantillon de mélasse distincte est relative à une levure de référence obtenue lorsque ladite levure de référence est alimentée avec ledit échantillon de mélasse distincte. Dans un ou plusieurs modes de réalisation, la qualité de mélasse d'un échantillon de mélasse distincte est définie par au moins un score de qualité de mélasse, ledit au moins un score de qualité de mélasse étant relatif à ladite au moins une performance de levure.

Par levure de référence, il peut être entendu une levure de référence utilisée dans un protocole de qualification ayant déterminé au moins une performance de ladite levure de référence, ladite levure de référence ayant été alimentée avec ledit échantillon de mélasse distincte.

Les caractéristiques exposées dans les paragraphes suivants peuvent, optionnellement, être mises en œuvre. Elles peuvent être mises en œuvre indépendamment les unes des autres ou en combinaison les unes avec les autres.

Selon un ou plusieurs modes de réalisations, ladite au moins une performance est basée sur au moins un critère choisi parmi :
- une capacité de multiplication de la levure alimentée par ladite mélasse courante,
- une capacité de levée de pain,
- une capacité à se conserver,
- une capacité à être séchée,
- une capacité à fixer des éléments structurels,
- une capacité à assimiler les nutriments,
- une capacité à s'adapter à des stress.

Par capacité de multiplication de la levure alimentée par ladite mélasse courante, il peut être entendu un rendement de production par unité de masse de mélasse courante mise en œuvre ou un ratio quantité de levure produite par quantité de sucre engagée dans le procédé de production.

Par éléments structurels, il peut être entendu des éléments tels que l'azote, le phosphore, le carbone, l'hydrogène, l'oxygène ou encore le souffre.

Dans un ou plusieurs modes de réalisation, la qualité de la mélasse courante déterminée par ledit modèle statistique est définie par au moins un score de qualité de mélasse, ledit au moins un score de qualité de mélasse étant relatif à ladite au moins une performance.

Dans un ou plusieurs modes de réalisation, plutôt que l'utilisation d'un score ou en combinaison avec un score, la qualité de la mélasse courante déterminée par ledit modèle statistique peut être définie par une classe telle que bonne, mauvaise, moyenne, très bonne, feu vert pour utiliser cette mélasse courante, feu rouge pour rejeter, ou modifier cette mélasse courante, etc., la classe étant relative à au moins une performance de la levure obtenue lorsqu'elle est alimentée avec cette mélasse courante.

Dans un ou plusieurs modes de réalisation, le procédé peut comprendre en outre :
- illuminer un échantillon de mélasse par un premier signal lumineux émis interagissant avec l'échantillon, et collecter un deuxième signal lumineux , résultant de l'interaction entre le premier signal lumineux et l'échantillon courant ; et
- mesurer le deuxième signal lumineux, dans des bandes de fréquences choisies, pour établir un spectre optique représentatif d'une signature chimique de l'échantillon courant de mélasse.

Dans un ou plusieurs modes de réalisation, le spectre optique établi et les spectres optiques de référence peuvent être mesurés dans des conditions similaires d'illumination et de mesure de signaux lumineux.

Ainsi par exemple, dans un mode de réalisation, le modèle statistique peut être déterminé par la mise en œuvre d'un apprentissage automatique, par exemple à partir d'une forêt aléatoire pour parvenir à un ensemble d'arbres de décision définissant le modèle statistique.

Dans un ou plusieurs modes de réalisation, le spectre optique peut comprendre un ensemble de nombre d'ondes, un ou plusieurs nombres d'ondes de l'ensemble de nombres de d'onde étant représentatifs d'un composé chimique de la mélasse.

Selon un ou plusieurs modes de réalisation, la mélasse peut comporter des composés parmi au moins un sucre, un minéral, une vitamine, et le spectre optique comporte une pluralité de nombres d'onde, et dans lequel au moins un nombre d'onde caractérise la présence d'un desdits composés.

Selon un ou plusieurs modes de réalisation, ladite au moins une caractéristique spectrale peut être déterminée selon au moins un critère choisi parmi une présence ou une absence d'un nombre d'onde prédéfini, une position d'un nombre prédéfini, une valeur d'intensité d'un nombre d'onde prédéfini, un dépassement de seuil d'une amplitude d'un nombre d'onde prédéfini, une valeur maximale ou minimale d'intensité d'un nombre d'onde prédéfini, un écart d'intensité entre deux nombres d'onde prédéfini, une intensité d'un nombre d'onde prédéfini comprise entre deux valeurs d'intensité prédéterminées, ou encore des combinaisons simples ou complexes des caractéristiques spectrales précitées.

Dans un ou plusieurs modes de réalisations, ladite au moins une caractéristique spectrale peut être déterminée selon au moins un critère choisi parmi une présence ou une absence d'une raie prédéfinie, une position d'une raie prédéfinie, une valeur d'amplitude de raie prédéfinie, un dépassement de seuil d'une amplitude de raie prédéfinie, une valeur maximale ou minimale d'amplitude de raie prédéfinie, un écart d'amplitude entre deux raies prédéfinies, une amplitude d'une raie comprise entre deux valeurs d'amplitude prédéterminées, ou encore des combinaisons simples ou complexes des caractéristiques spectrales précitées.

Selon un ou plusieurs modes de réalisation, la mesure optique peut être réalisée par au moins une technique parmi une spectroscopie RAMAN, une spectroscopie IR, ou une spectroscopie THZ.

Selon un ou plusieurs modes de réalisation, la plage de valeurs des spectres optiques de références peut être comprise entre 1 cm⁻¹ et 2500 cm⁻¹.

Selon un ou plusieurs modes de réalisation, la plage de valeurs des spectres optiques de références peut être comprise entre 436 cm⁻¹ et 1700 cm⁻¹.

Selon un ou plusieurs modes de réalisation, la mélasse est une mélasse de canne ou une mélasse de betterave ou un mélange des deux, ou un mélange de produits sucriers tels qu'une mélasse et un sirop de sucre, ou un mélange de produits dont un des composants est la mélasse.

La présente divulgation porte aussi sur un programme d'ordinateur, selon la revendication 12, comprenant des instructions de code de programme pour l'exécution du procédé de la présente divulgation lorsque le programme peut être exécuté sur un ordinateur.

La présente divulgation concerne aussi un support d'enregistrement non transitoire lisible par un ordinateur, selon la revendication 13, sur lequel est enregistré un programme pour la mise en œuvre du procédé de la présente divulgation lorsque ce programme est exécuté par un processeur.

La présente divulgation porte aussi sur un procédé, selon la revendication 14, de production de levures par fermentation pouvant comprendre :
- déterminer une qualité de mélasse respective pour chaque mélasse d'une pluralité de mélasses fournies en entrée de la réaction de fermentation selon le procédé de qualification de mélasse décrit dans la présente divulgation, et
- alimenter la réaction de fermentation avec chaque mélasse de la pluralité de mélasses dont la qualité de mélasse respective est supérieure à une qualité prédéterminée, et pour toute ou partie des mélasses présentant une qualité de mélasse inférieure à la qualité prédéterminée, alimenter la réaction de fermentation après modification de la mélasse, ou/et rejeter la mélasse.

Par modification de la mélasse, dans un ou plusieurs modes de réalisation, on peut envisager au moins une modification choisie parmi:
- mélanger la mélasse présentant une qualité de mélasse inférieure à la qualité prédéterminée, avec une autre mélasse présentant une qualité de mélasse distincte, et de sorte que le mélange de mélasses présente une qualité de mélasse supérieure à la qualité prédéterminée,
- ajouter un ou plusieurs adjuvants de sorte que la mélasse améliorée par les adjuvants présente une qualité de mélasse supérieure à la qualité prédéterminée.

La qualité de mélasse respective de chaque mélasse de la pluralité de mélasses et qui est relative à au moins une performance de levure obtenue peut être déterminée pour des conditions de fermentations identiques (e.g. température et pression du fermenteur, type de fermenteur, etc.) et pour une même souche de levure.

### Brève description des dessins

D'autres caractéristiques, détails et avantages apparaîtront à la lecture de la description détaillée ci-après, et à l'analyse des dessins annexés, sur lesquels :
**Fig. 1**

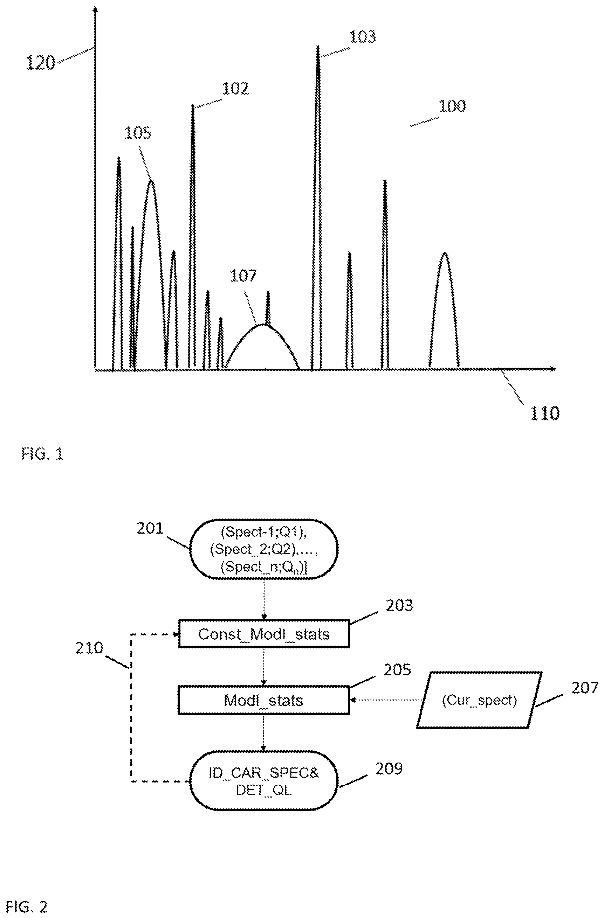

   [Fig. 1] décrit de manière schématique, dans un ou plusieurs modes de réalisation, un exemple de spectre optique d'une mélasse.
**Fig. 2**
   [Fig. 2] illustre un, procédé pour déterminer une qualité d'une mélasse à partir d'une mesure optique dans un ou plusieurs modes de réalisation.
**Fig. 3**

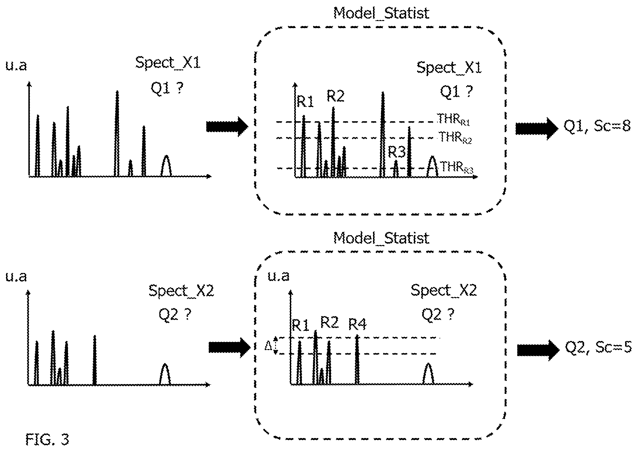

   [Fig. 3] illustre un exemple d'application du modèle statistique obtenu selon la méthode de la présente divulgation sur des spectres de mélasses inconnues.
**Fig. 4a**

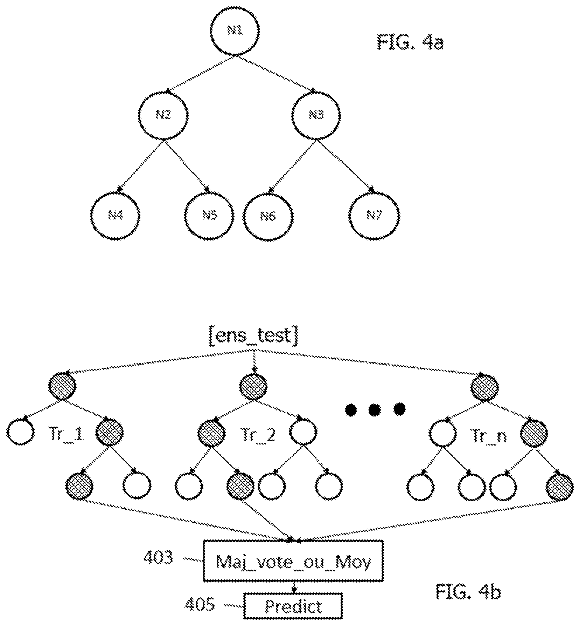

   [Fig. 4a] illustre le principe de forêt aléatoire pour un modèle statistique dans un ou plusieurs modes de réalisation.
**Fig. 4b**
   [Fig. 4b] illustre, le principe de forêt aléatoire pour un modèle statistique dans un ou plusieurs modes de réalisation.
**Fig. 5**

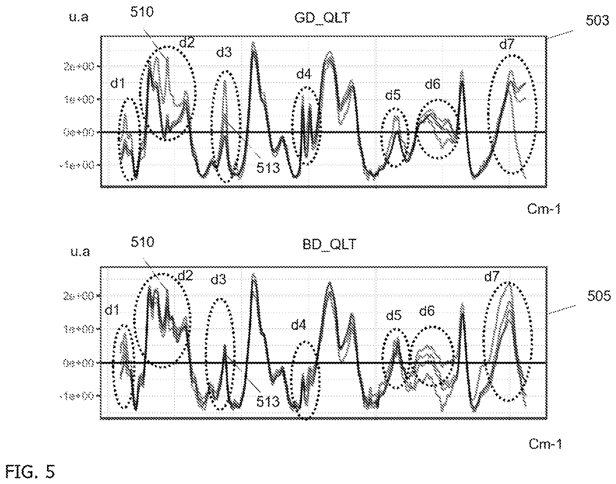

   [Fig. 5] présente différents spectres de mélasses de canne de qualité connue obtenus par spectroscopie RAMAN.
**Fig. 6**

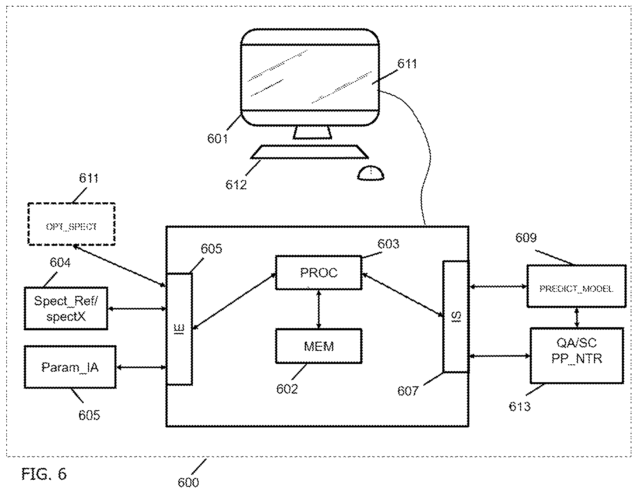

   [Fig. 6] présente un dispositif pour mettre en œuvre le procédé de la présente divulgation selon un ou plusieurs modes de réalisation.

### Description des modes de réalisation

Les termes « pic » et « raie » peuvent être interprétés de la même manière et sont interchangeables dans la présente divulgation. De même les termes « intensité » et « amplitude » peuvent être interprétés de la même manière et sont interchangeables dans la présente divulgation.

La figure 1 décrit de manière schématique, dans un ou plusieurs modes de réalisation, un exemple de spectre optique d'une mélasse.

Il existe de nombreuses techniques de mesure optique permettant d'obtenir un spectre optique, comme par exemple la spectroscopie infrarouge, micro-ondes, Térahertz, ou encore la spectroscopie RAMAN. Toutes ces techniques reposent sur un même principe consistant à envoyer une onde électromagnétique (i.e. des photons) sur la matière à sonder en faisant varier la fréquence de l'onde. Une onde résultante de l'interaction onde-matière peut ensuite être collectée, soit en transmission soit en réflexion, pour chaque fréquence de l'onde électromagnétique. À partir de l'onde résultante, il est alors possible de déterminer un spectre optique représentatif des constituants d'une matière organique (e.g. une mélasse), c'est-à-dire une signature chimique de la mélasse.

Un spectre optique 100 peut être représenté comme un ensemble de pics, où chaque pic peut être associé à une position (p. ex. abscisse) et une amplitude (e.g. ordonnée) dans le spectre. L'abscisse 110 d'un spectre optique peut être exprimée soit en fréquence (Hz), soit en longueur d'onde ou soit en nombre d'onde (cm-1). L'ordonnée 120 peut être exprimée dans une unité arbitraire qui peut être fonction du traitement de signal appliqué sur les données de mesure. En effet, afin de pouvoir être correctement exploitable, il est courant qu'un ou plusieurs traitements de données soient appliqués sur les données brutes (i.e. des données issues des mesures optiques) afin de permettre la comparaison entre les spectres par exemple.

À titre d'exemple, dans le cas de spectroscopie RAMAN, une ligne de base, une dérivation de cette ligne de base ou encore une combinaison avec la normalisation des données peuvent être réalisées afin de supprimer certains pics présentant des aberrations ou dont la présence résulte du Laser utilisé pour la spectroscopie,

A chaque technique de mesure optique peut correspondre une plage spécifique de fréquences (ou longueurs d'onde ou nombre d'onde) d'utilisations dans laquelle la fréquence de l'onde incidente varie. Par exemple, la plage de longueurs d'onde (exprimée en microns) peut être comprise entre [1µ m-20µm], [0.3µ m-0.7µm], ou encore [1 cm⁻¹ - 2500cm⁻¹]. Dans un ou plusieurs modes de réalisation, cette plage de fréquence peut être située entre [436 cm⁻¹ - 1700 cm⁻¹].

En fonction de la technique de mesure optique et de la plage de longueur d'ondes utilisées pour obtenir un spectre d'une matière organique, la position d'un pic peut correspondre, par exemple, soit à une liaison atomique ou chimique, soit à un groupement fonctionnel d'une molécule organique, ou encore les vibrations d'une molécule. Plusieurs positions de pic peuvent donc correspondre à la présence d'une même molécule (ou constituant, par exemple une vitamine) dans une matière organique en reflétant, par exemple les liaisons chimiques ou groupements fonctionnels de cette molécule

L'amplitude d'un pic peut avoir différentes significations en fonction de la technique de mesure optique utilisée et des traitements de données appliqués sur les données optiques brutes.

Les techniques de mesure optique permettant de réaliser la spectroscopie d'un milieu sont généralement complémentaires, et un même milieu peut être mesuré par différentes techniques de mesure optique (p. ex. différents types de spectromètres) afin d'obtenir une cartographie relativement complète de ce milieu.

En fonction des techniques de mesure utilisées, l'exploitation de spectres peut être plus ou moins compliquée que ce soit visuellement ou selon les méthodes classiques d'exploitations de données de spectres. En effet, un spectre optique peut comprendre une multitude de pics (ou raies), certains pouvant être parfaitement discrétisés 102 ;103, tandis que d'autres peuvent être tellement rapprochés qu'ils forment un ensemble 105 ;107 rendant difficile leur exploitation. En outre, en fonction de la résolution utilisée (p. ex. le pas de la mesure optique), un pic en fonction de sa largeur à mi-hauteur peut s'étaler de manière plus ou moins importante sur spectre optique, et ainsi pouvant compliquer un peu plus l'interprétation des données.

En outre, alors qu'un changement de la technique de mesure optique utilisée peut paraitre évident lorsque le spectre optique obtenu n'est pas exploitable (p. ex. amas de raies 107), l'utilisation d'une autre technique de mesure optique peut conduire à l'obtention d'un spectre optique complètement ou partiellement différent du spectre obtenu initialement. En effet, les composées/molécules recherchées peuvent ne pas être sensibles aux longueurs d'onde utilisées par une technique optique alternative.

Ainsi, il peut être donc crucial d'être en capacité de pouvoir exploiter un spectre optique, indépendamment de la configuration des pics du spectre, et plus particulièrement quand des interprétations et des comparaisons entre un nombre important de spectres optiques obtenus à partir d'échantillons relativement proches dans leur empreinte chimique/moléculaire sont recherchées.

La figure 2 illustre un procédé pour déterminer une qualité d'une mélasse à partir d'une mesure optique dans un ou plusieurs modes de réalisation.

À partir de spectres optiques (Spect_ref1... Spect_refn, n'étant un entier positif) 201 de référence d'échantillons de mélasses distinctes qui sont connues et qui sont associées respectivement à des qualités de mélasse connues (Q1, Q2, Qₙ), un modèle statistique de qualification 203 de mélasse en fonction d'au moins une caractéristique spectrale des spectres de référence peut être construit par apprentissage automatique.

L'apprentissage-automatique peut consister à déterminer, dans les spectres de références de mélasses, des configurations spectrales amenant à une certaine qualité de mélasse, en particulier certaines qualités de mélasse propres à l'obtention d'une ou plusieurs performances recherchées pour une levure par exemple.

Sur la base des configurations spectrales déterminées, l'apprentissage-automatique peut alors construire une ou plusieurs règles qui, ensemble(s), constituent un modèle statistique corrélant une ou plusieurs caractéristiques spectrales du spectre à une qualité de mélasse, cette qualité de mélasse étant relative à une ou plusieurs performances d'une levure produite en utilisant cette qualité de mélasse.

Les spectres de références et les qualités respectives des mélasses associées aux spectres de références peuvent correspondre à des données d'entrée pour alimenter l'apprentissage-automatique, et chaque spectre de référence peut être représentatif d'une structure (e.g. chimique) d'une mélasse (par exemple, de canne ou de betterave). En outre, chaque qualité de mélasse présentée en données d'entrée et associée respectivement à un spectre de référence peut être relative à au moins une performance de levure (e.g. levure de référence) obtenue lorsque cette levure est alimentée avec cette mélasse.

En outre, dans un ou plusieurs modes de réalisation, la qualité de mélasse peut être définie par au moins un score respectif Scₙ ou/et selon une classe de mélasse (e.g. bonne, mauvaise, moyenne, feu rouge, feu vert etc.) ou/et une corrélation entre son profil spectral et un groupe statistique de performances industrielles.

Dans un ou plusieurs modes de réalisation, le score de qualification de mélasse peut être relatif à l'impact de la qualité d'une mélasse, lorsque celle-ci est utilisée comme source de nutriments dans une production de levure, sur la ou les performances de la levure obtenue.

Par exemple, le score de chaque mélasse associée à un spectre de référence peut avoir une valeur comprise entre 0 et 1 (e.g. valeur 0 = mauvais et valeur 1 = bonne mélasse) ou 1 et 10. La valeur 1 peut être représentative d'une mélasse de mauvaise qualité et la valeur 10 représentative d'une mélasse de très bonne qualité. Ainsi, un score (ou une valeur de score) supérieure à 8 peut correspondre à une mélasse permettant d'obtenir une levure avec de très bonnes performances, par exemple une très bonne capacité de levée de pâte boulangère comme du pain et/ou une très bonne capacité de multiplication (i.e. un bon rendement de production de quantité de levure).

Dans un ou plusieurs modes de réalisation, une qualité de mélasse peut être définie selon une plage de valeurs. Par exemple, un score compris entre 0 et 3 peut correspondre à une mélasse de mauvaise qualité, un score compris entre de 3 et 5 peut correspondre à une mélasse de moyenne qualité, un score compris entre 5 et 8 peut correspondre à une mélasse de bonne qualité, et un score supérieur à 8 sur une échelle de 10 peut correspondre à une mélasse de très bonne qualité.

Selon un autre exemple, dans un ou plusieurs modes de réalisation, la qualité de mélasse peut être définie par une pluralité de scores, chaque score étant relatif à une performance respective. Par exemple, un spectre optique de référence peut être associé à un premier score relatif à une capacité de levée de pain, et à un deuxième score relatif à une capacité de multiplication (i.e. rendement de production de levure).

Les scores peuvent être combinés entre eux afin d'en tirer des informations exploitables par le personnel en charge de la production de la levure au sein d'une industrie ou d'un laboratoire d'essais.

Le score de chaque mélasse associée à un spectre de référence peut avoir été déterminé préalablement sur la base d'un ou plusieurs protocoles de qualification.

À titre d'exemple, un protocole de qualification d'une mélasse peut être la mise en œuvre de cette mélasse avec une levure de référence afin de déterminer les performances de la levure de référence, en matière de multiplication cellulaire et d'activité enzymatique.

Par exemple, la performance basée (ou relative à) sur la capacité de levée de pain peut être évaluée par la capacité de la levure préparée (e.g. levure de référence préparée) à produire du gaz carbonique, ou/et peut être évaluée par l'activité fermentative de la levure.

L'activité fermentative est déterminée par mesure du dégagement gazeux en dioxyde de carbone (exprimé de manière absolue en volume ou de manière relative en pourcentage) d'un pâton donné à l'aide par exemple d'un fermentomètre de Burrows et Harrison décrit dans la « Journal of Institute of Brewing », vol. LXV, No. 1, janvier-février 1959 ou d'un Risograph selon le protocole décrit par Rattin et al., 2009 (Cereal Foods World, 54 (6) : 261-265).

Dans un ou plusieurs modes de réalisation, un score Scₙ de qualité de mélasse supérieur à 5 (i.e. correspondant à une mélasse de bonne qualité par exemple), peut être relatif à une capacité de levée de pain (i.e. une performance de levure) en comparaison de celle généralement observable sur les levures produites dans un procédé de référence. De même, un score Scₙ de qualité de mélasse inférieur à 3 (i.e. correspondant à une mélasse de mauvaise qualité), peut être relatif à une capacité de levée de pain permettant d'atteindre des valeurs de volumes spécifiques inférieures aux valeurs généralement observables sur les levures produites dans un procédé de référence.

La capacité de multiplication de la levure alimentée par une mélasse courante, également appelé rendement de production, peut être exprimée en quantité de levure produite par quantité de sucre engagé, ou par la quantité de sucre engagé par quantité de levure produite.

Dans un ou plusieurs modes de réalisation, un score Scₙ de qualité de mélasse supérieur à 5 (i.e. correspondant à une mélasse de bonne qualité par exemple), peut être relatif à une capacité de multiplication de la levure (i.e. une performance de levure) généralement observable sur les levures produites dans un procédé de référence. De même, un score Scₙ de qualité de mélasse inférieur à 3 (i.e. correspondant à une mélasse de mauvaise qualité), peut être relatif à une capacité de multiplication de la levure permettant d'atteindre valeurs inférieures aux valeurs généralement observables sur les levures produites dans un procédé de référence.

Dans un ou plusieurs modes de réalisation, les données d'entrées des spectres de référence pour l'apprentissage automatique peuvent être sous la forme d'un ensemble de nombres d'onde respectivement associés à une valeur d'intensité (ou amplitude). Par, exemple, l'ensemble de nombres d'onde peut être compris entre 0 et 2000 cm⁻¹ et selon un pas de nombre d'onde de 0,1 cm⁻¹.

Il existe de nombreuses techniques d'apprentissage automatique. Toutes les méthodes d'apprentissage automatique peuvent être utilisées pour apprendre les règles liant les caractéristiques des mélasses et la performance des levures. Dans un ou plusieurs modes de réalisation, l'apprentissage automatique peut notamment être basé sur l'utilisation des forêts aléatoires (« random forest »), sur l'utilisation d'un ou plusieurs algorithmes de régression linéaire, sur l'utilisation des K plus proches voisins, sur l'utilisation de l'apprentissage profond (Deep learning), ou encore l'utilisation de réseaux de neurones.

En outre, l'apprentissage automatique peut comprendre, en données d'entrée, un ou plusieurs hyperparamètres. Un hyperparamètre est un paramètre utilisé pour paramétrer l'algorithme (e.g. random forest, deep learning, etc.) avant tout apprentissage. Les hyperparamètres peuvent être propres à un type d'apprentissage automatique et peuvent, par conséquent, varier en fonction du type d'apprentissage automatique utilisé.

À titre d'exemple, un hyperparamètre peut être le nombre d'arbres de décision composant une forêt aléatoire, le nombre de nœuds dans un arbre, la taille des nœud finaux, ou encore le nombre d'observations choisies par « bootstrap » pour construire chaque arbre.

Après la détermination du modèle, celui-ci peut ensuite être utilisé pour identifier 205, pour un spectre courant d'un échantillon de mélasse courante en données d'entrée 207, au moins une caractéristique spectrale du spectre courant, et déterminer une qualité 209 de la mélasse courante.

A partir de la qualité déterminée de la mélasse courante, une ou plusieurs performances d'une levure produite en utilisant la mélasse courante comme source de nutriment peuvent être déterminées. La qualité de la mélasse courante peut être représentée suivant une classe (e.g. mauvaise, moyenne, bonne, très bonne, feu vert lorsqu'elle peut être utilisée ou feu rouge lorsqu'elle ne peut pas être utilisée ou qu'elle nécessite d'être modifiée, etc.) ou/et suivant un ou plusieurs scores relatifs à des performances de la levure, ou/et une corrélation entre son profil spectral et un groupe statistique de performance industrielle.

Bien entendu, la performance de levure obtenue lorsque la levure est alimentée avec la mélasse courante dépend de plusieurs contributions telles que la souche de levure utilisée pour produire la levure, des conditions de productions (e.g. pression, température dans le fermenteur, configuration du fermenteur, etc.), et des entrants parmi lesquels la mélasse utilisée pour alimenter la production de levure. Dans le cadre du procédé de la présente divulgation, il est entendu que la ou les performances de levure déterminées en fonction de qualités de mélasse sont déterminées pour une souche de levure identique, des conditions de production identique, et entrants identiques (à l'exception de la mélasse).

Dans un ou plusieurs modes de réalisation, chaque spectre de référence d'une mélasse respective fourni pour l'apprentissage- automatique peut avoir été obtenu par caractérisation de cette mélasse respective selon une ou plusieurs techniques de mesure optique. Par exemple, chaque spectre de référence peut être représentatif d'une combinaison de spectres optiques d'une même mélasse et obtenus par une technique de mesure optique respective.

Dans un ou plusieurs modes de réalisation, la technique de mesure optique sur les mélasses permettant d'obtenir les spectres de référence est la même que celle utilisée sur la mélasse courante pour obtenir le spectre courant. En outre, dans un ou plusieurs modes de réalisation, la technique de mesure optique utilisée pour obtenir les spectres peut être la spectroscopie RAMAN.

Dans un ou plusieurs modes de réalisation, les données obtenues après la détermination d'une qualité de mélasse relative à une performance de levure suivant le spectre d'entrée 207 peuvent être utilisées pour alimenter 210 l'apprentissage automatique. Ainsi, avantageusement, le modèle statistique peut être amélioré de manière continue.

Bien entendu, le modèle statistique peut ne pas être construit à chaque fois qu'il est demandé de qualifier une mélasse inconnue. Le modèle statistique peut être déterminé une seule fois pour être ensuite utilisé avec des spectres inconnus de mélasses.

La méthodologie de la forêt aléatoire (« random forest ») est décrite de manière plus détaillée dans la publication suivante :
- Leo Breiman, Random Forests , Machine Learning, vol. 45, no 1, 2001, p. 5-32

Comme décrit précédemment, la présente divulgation ne se limite pas à l'utilisation de forêt aléatoire. D'autres types d'apprentissage automatique peuvent également convenir, comme par exemple les méthodes d'apprentissage automatique décrites dans les publications suivantes :
- The Elements of Statistical Learning, 2nd edition, Trevor Hastie, Robert Tibshirani, Jerome Friedman, ISBN-13: 978-0387848570
- Deep Learning , lan Goodfellow, Yoshua Bengio et Aaron Courville, ISBN-13: 978-0262035613*.*

La figure 3 illustre un exemple d'application du modèle statistique obtenu selon la méthode de la présente divulgation sur des spectres de mélasses inconnues.

La qualité respective (Q1 ; Q2) de deux mélasses M1 et M2 pouvant potentiellement être utilisées comme source de nutriments dans une production de levure (e.g. production de levure par fermentation) peut être recherchée.

Cette qualité peut être recherchée afin de savoir si les mélasses M1 et M2 peuvent être utilisées pour alimenter une production de levure tout en permettant d'atteindre une ou plusieurs performances cibles de la levure produite, ou/et afin de déterminer la mélasse la plus adaptée pour produire une levure présentant une ou plusieurs performances cibles, ou/et selon un compromis d'une ou plusieurs performances cibles.

En référence à la figure 3, à titre d'exemple, une discrétisation entre les deux mélasses M1 et M2 peut être recherchée afin de déterminer la mélasse pouvant amener à l'obtention d'un rendement de production de levure supérieur à une valeur généralement observée avec une levure de référence opérée dans un procédé de référence et/ou l'obtention d'une levure (produite) qui permet d'atteindre des valeurs de volumes spécifiques supérieures à une valeur généralement observée avec une levure de référence opérée dans un procédé de référence. Selon un autre exemple, la discrétisation entre les deux mélasses M1 et M2 peut être recherchée afin de déterminer la mélasse qui présente le rapport de performances recherché, e.g. un rendement de production / une capacité de levée de pain.

Chaque mélasse M1 ; M2 peut être associée respectivement à un spectre optique (Spect_X1 et Spect_X2), obtenu par exemple par spectroscopie RAMAN.

Ainsi, l'application du modèle statistique (Model_statist) de qualités de mélasse sur les spectres optiques de mélasse (Spect_X1 et Spect_X2), différents des spectres de référence ayant servi à l'apprentissage automatique, peut permettre d'identifier des caractéristiques spectrales particulières. Par exemple, le modèle statistique, peut déterminer une qualité Q1 (et/ou un score et/ou une classe) du spectre Spect_X1 sur la base de l'identification des caractéristiques spectrales correspondant à la présence des nombres d'onde R1, R2 et R3 couplés à un seuil respectif d'amplitude TH_{R1}, TH_{R2}, TH_{R3} pour chacun des nombres d'onde. De même, le modèle de prédiction peut déterminer une qualité Q2 du spectre Spect_X2 sur la base de l'identification des caractéristiques spectrales correspondant à la présence des nombres d'onde R1, R2 et R4 couplé à une plage d'amplitude Δ à respecter pour les nombres d'onde R1 et R2.

A partir des qualités déterminées (e.g. suivant un score), la mélasse M1 peut sembler être une meilleure candidate pour alimenter une production de levure que la mélasse M2. En effet, la mélasse M1 présente un score de 8, représentatif d'une très bonne mélasse, c'est-à-dire permettant, par exemple, l'obtention d'un rendement de production de levure supérieur à une valeur généralement observée avec une levure de référence opérée dans un procédé de référence, et/ou ou l'obtention d'une bonne capacité de levée de pain définie par des valeurs de volumes spécifiques atteignables supérieures à une valeur généralement observée avec une levure de référence opérée dans un procédé de référence.

Dans un ou plusieurs modes de réalisation, pour une pluralité de mélasses pouvant être potentiellement utilisées pour alimenter une production de levure (par fermentation), les mélasses présentant une qualité de mélasse supérieure à une qualité prédéterminée (e.g. un ou plusieurs scores) peuvent être utilisées pour alimenter une production de levure. Dans un ou plusieurs modes de réalisation, parmi les mélasses ayant une qualité supérieure à une qualité prédéterminée, la priorité pour alimenter la production de levure peut être donnée à la mélasse présentant la meilleure qualité. À titre d'exemple, la meilleure qualité peut être déterminée selon un ordre croissant ou décroissant des scores de qualité des mélasses ou selon un ordre croissant ou décroissant des moyennes des scores respectifs de qualité de chaque mélasse.

Les figures 4a et 4b illustrent le principe de forêt aléatoire pour un modèle statistique dans un ou plusieurs modes de réalisation.

Le principe de l'algorithme de forêt aléatoire est de déterminer, selon un vote à la majorité pour des données catégoriques ou selon une moyenne de résultats individuels pour des données quantitatives, la prédiction la plus probable réalisée par une multitude d'arbres de décision constituant la forêt aléatoire.

Tous les arbres de décision d'une forêt aléatoire peuvent être indépendants les uns des autres, et la prédiction déterminée par chaque arbre de décision avant le vote à la majorité ou l'établissement de la moyenne peut être décorrélée des prédictions déterminées par les autres arbres de décision de la forêt aléatoire.

Une première étape de cet algorithme peut consister à construire des arbres de décisions (ou arbres décisionnels). La figure 4a présente un tel arbre de décision pouvant, par exemple, être divisé en trois parties :
- un nœud racine N1 correspondant au premier nœud de l'arbre (i.e. nœud d'entrée),
- des nœuds internes N2 ; N3 pouvant présenter des descendances (i.e. d'autres nœuds),
- des nœuds terminaux N4 ; N5 ; N6 ; N7 (aussi appelés feuilles).

Chaque "nœud" intermédiaire, c'est-à-dire les nœuds N2 et N3, peut réaliser un test portant sur une variable dont le résultat indique la branche à suivre dans l'arbre (i.e. N4, N5, N6 ou N7). Ainsi, le ou les tests effectués à un nœud peuvent définir une règle de partition par exemple.

Les nœuds N1 à N3 peuvent être définis par des variables explicatives (e.g. de type quantitatif ou qualitatif).

Afin de construire les différents arbres de décision tels que celui présenté en figure 4a, une sélection de sous-ensembles d'individus à partir du jeu de données d'entrainement avec remises peut être effectuée grâce à un échantillonnage dit « bootstrap », et qui consiste à effectuer des tirages (avec remise) de manière aléatoire d'individus dans le jeu de données d'entrainement.

Par remise, il peut être entendu que les candidats sélectionnés dans un sous-ensemble restent dans le jeu d'entrainements, c'est-à-dire qu'ils peuvent être sélectionnables pour un autre sous-ensemble. Les individus utilisés pour construire un arbre de décision sont généralement appelés « in bag » et les individus utilisés pour évaluer la prédictivité de l'arbre sont généralement appelés « out of bag » (dit « OOB »).

Ce jeu de données d'entrainement peut être lui-même obtenu suivant la mise en place d'une stratégie de validation croisée consistant à séparer le jeu de données initial (e.g. 70/30) en deux sous-jeux de données, l'un pour l'entrainement (e.g. 70%) et l'autre pour tester (e.g. 30%) le modèle de prédiction.

Chaque arbre de décision peut ensuite être construit (ou entrainé) sur un sous-ensemble respectif d'individus. C'est-à-dire qu'il peut y avoir autant de sous-ensembles d'individus qu'il y a d'arbres de décision, et la construction de l'arbre n'est effectué que sur un seul sous-ensemble d'individus.

Dans le cas de la présente divulgation, le jeu de données initial peut correspondre à un ensemble de spectres optiques de références (e.g. sous forme d'ensembles de nombres d'onde) associés à une qualité respective de mélasse (ou/et un ou plusieurs scores respectifs de mélasse) pouvant être relative à au moins une performance de levure (e.g. levure de référence) obtenue lorsque la levure est alimentée avec cette mélasse, et la variable respective utilisée dans le test de chaque nœud N1 à N3 peut être, par exemple, une intensité (ou amplitude) d'un nombre d'onde spécifique.

Au cours de l'entrainement (ou construction de l'arbre), à chaque construction d'un nouveau nœud, la variable permettant la partition au niveau du nœud (en deux nœuds descendants) peut être déterminée par l'algorithme de l'apprentissage automatique à partir du sous-ensemble aléatoire issu du jeu de données d'entrainement. Par exemple, l'apprentissage automatique peut passer en revue (e.g. suivant le pas de discrétisation des spectres optiques) l'ensemble (ou un sous-ensemble) des nombres d'onde des spectres optique de références du sous-ensemble aléatoire afin de déterminer un nombre d'onde et/ou une intensité à utiliser comme variable. Plus précisément, l'algorithme peut chercher à déterminer s'il existe, par exemple, une valeur d'intensité permettant de séparer les spectres de façon la plus pure entre les spectres correspondant à des mélasses de bonne qualité ou de mauvaise qualité. À titre d'exemple, le test associé au nœud N2 peut être de déterminer si l'amplitude (ou intensité) associée à un nombre d'onde particulier est supérieure à un seuil d'intensité ou compris dans une plage d'intensité.

Le choix d'une variable plutôt qu'une autre peut être basé sur la mesure de la pureté du nœud en utilisant, par exemple, l'indice de Gini ou l'entropie de Shannon.

Ainsi, aucune des données d'entrée fournies pour l'apprentissage automatique ne vient l'influencer dans son choix des variables pour le partitionnement à chaque nœud. L'apprentissage automatique par forêt aléatoire peut déterminer ici ses propres variables (e.g. nombre d'ondes, amplitudes, etc.) afin de construire un modèle de statistique, sans a priori sur la présence d'un ou plusieurs nombres d'onde qui devraient être détectés dans les spectres de références. Par exemple, le nœud N3 peut comprendre un test basé sur une valeur d'intensité d'un nombre d'onde ne caractérisant aucune raie spécifique ou la présence d'éléments en particulier (e.g. vitamine, nutriments, etc.) mais ayant été déterminé simplement comme le nombre d'onde avec sa valeur d'intensité permettant une partition la plus pure.

Les nœuds terminaux N4 à N7 correspondent quant à eux à des qualités (ou/et scores) respectives de mélasse et qui peuvent être relatives aux performances possibles d'une levure.

Pour chaque arbre de de décision, une erreur de prédiction (erreur OOB) peut être calculée afin de déterminer le pourcentage d'individus mal classés par l'arbre de décision. Plus ce taux est faible et plus l'arbre de décision peut être considéré comme de bonne qualité dans sa prédiction.

Comme décrit précédemment, la plupart des méthodes d'apprentissage automatique permettent d'identifier les facteurs importants qui influencent le plus les prédictions ou les classifications. Dans le cadre des forêts aléatoires, l'algorithme peut permettre de déterminer quelles intensités ou nombre d'onde spécifiques sont importants pour discriminer les mélasses. Plusieurs méthodes d'évaluation ont été décrites dans la littérature. Par exemple, cette importance peut être déterminée par le nombre de fois où une valeur d'intensité associée à un nombre d'onde est utilisée pour partitionner les données au niveau d'un nœud dans les différents arbres de décision de la forêt aléatoire.

La détermination par le modèle statistique de certaines récurrences de valeurs d'intensités associées à un nombre d'onde donnée peut, par exemple, éventuellement renseigner sur la présence spécifique de molécule ou groupe de molécules comme des vitamines, des nutriments ou des sucres selon une certaine quantité (e.g. reflété par l'amplitude/intensité associée à ce nombre d'onde en spectroscopie RAMAN).

Par exemple, un test basé sur une valeur d'intensité d'un nombre d'onde pouvant caractériser une raie représentative d'un sucre spécifique peut être mise en œuvre par un nœud d'un ou plusieurs d'arbres de décision (de la forêt aléatoire), un autre test basé sur une valeur d'intensité d'un nombre d'onde pouvant caractériser une raie représentative d'un sucre spécifique peut être mise en œuvre par un nœud d'un ou plusieurs d'arbres de décision, etc.

De manière avantageuse, la détermination de certaines récurrences peut permettre de dégager une tendance sur les éléments importants particuliers permettant de discrétiser une bonne d'une mauvaise qualité de mélasse.

La figure 4b illustre un exemple de détermination d'une qualité de mélasse par le modèle statistique pour un spectre optique de référence en données d'entrée.

Un ou plusieurs spectres optiques de référence issu du jeu de données tests [ens_test] précédemment présentés peuvent être fournis en entrée du modèle statistique basé sur une forêt aléatoire entrainée comme décrit précédemment afin de tester la pertinence de ses prédictions en sortie de modèle.

Pour chaque spectre optique de référence fourni en entrée du modèle, les arbres Tr_1 ; Tr_2 ; Tr_n peuvent alors respectivement déterminer (nœuds grisés) une classe, c'est-à-dire un score ou/et une qualité de la mélasse du spectre de référence d'entrée. La détermination 405 de la qualité de mélasse peut ensuite être effectuée sur la base d'un vote à la majorité 403 ou selon la moyenne afin de déterminer la qualité et/ou le score de mélasse la plus probable. La qualité de mélasse du spectre de référence (e.g. spectre RAMAN, ou IR ou THz) fourni en entrée étant connue, puisqu'issue du jeu de données tests, la précision du modèle statistique basé sur la forêt aléatoire entrainée peut être déterminée en comparant la prédiction d'une qualité obtenue à partir du modèle entrainé avec la qualité réelle de la mélasse.

Le modèle statistique (Model_predict) présentant une précision de prédiction acceptable peut ensuite être utilisé pour déterminer une qualité et/ou un score d'une mélasse inconnue (e.g. Spect_X1 et spect_X2 de la figure 3) dont le spectre optique est fourni en entrée du modèle, et d'en déduire la ou les performances d'une levure produite en utilisant comme source de nutriment la mélasse inconnue.

La figure 5 présente, dans un ou plusieurs modes de réalisation, différents spectres de mélasses de canne de qualité connue obtenus par spectroscopie RAMAN.

Un spectre Raman peut être tracé en fonction du décalage Raman en nombre d'onde et de son intensité en unité arbitraire. Le décalage Raman correspond à la différence entre la lumière du laser monochromatique utilisé et la lumière diffusée. L'intensité Raman est le nombre de photons détectés issus d'une diffusion Raman Stokes. En effet, en spectroscopie Raman, il existe trois types de diffusions, diffusion de Rayleigh (élastique), diffusion Raman Stokes (inélastique), et diffusion Raman anti-Stoke (inélastique). En diffusion inélastique, le photon ayant été émis par un laser et ayant interagi avec de la matière n'est pas réémis à la même longueur d'onde, mais avec un décalage, Stokes ou anti-Stokes. La diffusion Raman Stokes est la plus communément utilisée pour obtenir le spectre optique d'un échantillon.

Ainsi, quatre spectres optiques (Stokes) 503 de mélasses de canne caractérisant une bonne qualité de mélasse (GD_QLT) et quatre spectres optiques (Stokes) 505 de mélasses de canne caractérisant une mauvaise qualité de mélasse (BD_QLT) sont présentés. Chaque spectre pouvant être représentatif d'une qualité de mélasse qui permet d'obtenir une levure avec au moins une performance respective (e.g. une certaine capacité de levée de pain et/ou une certaine capacité de multiplication de levure). Ces spectres optiques peuvent être, par exemple, des spectres de référence utilisés pour alimenter l'apprentissage automatique par forêt aléatoire décrit précédemment.

En référence à la figure 5, plusieurs zones de différences entre une bonne et une mauvaise qualité de mélasse (d1 à d7) peuvent être notables. Néanmoins, mis à part la zone d4 qui présente une différence marquée entre deux qualités de mélasse respectives, la plupart des autres zones identifiées ne permettent pas de déterminer clairement les critères importants pouvant discrétiser une bonne qualité de mélasse d'une mauvaise qualité de mélasse, c'est-à-dire une qualité de mélasse permettant d'obtenir une levure ayant une ou plusieurs performances acceptables. En effet, à partir des zones de différences d2 et/ou d3 (ou encore d6), certaines mélasses qui sont pourtant de qualité différente peuvent, cependant, présenter des spectres identiques ou très proches dans certaines zones 510 ; 513.

De plus, alors que la zone d4 semble présenter des différences marquées lorsque les mélasses sont de bonne ou de mauvaise qualité, les différences au niveau de cette zone peuvent être finalement peu ou pas pertinentes pour être représentatives d'une certaine qualité de mélasse (bonne ou mauvaise), et donc représentatives des performances d'une levure alimentée en production avec ces mélasses de certaine qualité.

Ainsi, alors qu'une analyse visuelle des spectres optiques rend très compliquée la recherche d'une corrélation entre une configuration d'une ou plusieurs caractéristiques spectrales et une qualité de mélasse permettant d'obtenir une levure ayant une ou plusieurs performances acceptables, l'utilisation avantageuse du procédé tel qu'il est décrit dans la présente divulgation, c'est-à-dire un apprentissage automatique couplée à des données d'entrées adéquatement choisies (p. ex. spectres de référence, hyperparamètres, etc.), permet d'obtenir un modèle statistique (e.g. basé sur une forêt aléatoire entrainée) de qualités de mélasse efficace dans sa prédiction de qualité de mélasses relatives à des performances de levure.

Comme mentionné précédemment, l'obtention d'un modèle statistique entrainé peut alors permettre, avant toute production de levure, de déterminer s'il convient d'écarter ou non cette mélasse pour cause de mauvaise qualité (e.g. score inférieur à 3 et/ou classe mauvaise et/ou feu rouge), c'est-à-dire ne permettant pas d'obtenir une levure ayant une ou plusieurs performances acceptables (e.g. selon un cahier des charges). En outre, à partir de la prédiction de la qualité ou du score de qualité de la mélasse, il peut être possible de déterminer avantageusement les nutriments et/ou les proportions de nutriments à utiliser de manière à compenser la mauvaise qualité d'une mélasse.

La figure 6 présente, dans un ou plusieurs modes de réalisation, un dispositif pour mettre en œuvre le procédé de la présente divulgation selon un ou plusieurs modes de réalisation.

Dans ce mode de réalisation, le dispositif 600 peut comprendre un ordinateur 601, cet ordinateur comprenant une mémoire 602 pour stocker des instructions de programme pouvant être chargées dans un circuit, et apte à amener le circuit 603 à exécuter le procédé de la présente divulgation lorsque les instructions de programme sont gérées par le circuit 603.

La mémoire 602 peut également stocker des données et des informations utiles pour réaliser le procédé de la présente description telles que décrites ci-dessus.

Le circuit 603 peut être par exemple:
- un processeur ou une unité de traitement apte à interpréter des instructions dans un langage informatique, le processeur ou l'unité de traitement peut comprendre, être associé ou être attaché à une mémoire comprenant les instructions, ou
- l'association d'un processeur / unité de traitement et d'une mémoire, le processeur ou l'unité de traitement adaptés pour interpréter des instructions dans un langage informatique, la mémoire comprenant lesdites instructions, ou
- une carte électronique dans laquelle l'enchainement du procédé est décrit dans du silicium, ou
- une puce électronique programmable telle qu'une puce FPGA (pour «Field-Programmable Gate Array»).

Cet ordinateur peut comprendre une interface d'entrée 605 pour la réception de données d'entrée utilisées pour l'apprentissage automatique et une interface de sortie pour fournir un ensemble de données utiles. À titre d'exemple, l'interface d'entrée peut recevoir des données d'entrée 604 telles que des spectres optiques de référence associés à une qualité de mélasse respective pour l'apprentissage automatique ou des spectres dont la qualité relative à au moins une performance d'une levure doit être déterminée. L'interface d'entrée peut aussi recevoir un ensemble de paramètres de configuration 605 pour configurer l'apprentissage automatique, par exemple des hyperparamètres. En outre, optionnellement, l'interface d'entrée peut être connectée 611 à un dispositif permettant la mesure optique (e.g. spectroscopie RAMAN), ou le dispositif 600 peut être directement intégré dans un dispositif de mesure optique.

À titre d'exemple, l'ensemble de données utiles fourni par l'interface de sortie 607 peut être un modèle de prédiction entrainé 609, par exemple un modèle statistique de qualité de mélasse relative à au moins une performance de levure obtenue à partir de cette mélasse. En outre, l'interface de sortie peut fournir une prédiction (ou détermination) 613 d'une qualité d'une mélasse (e.g. classe et/ou score) fournie 604 à l'interface d'entrée 605, la qualité de mélasse étant relative à au moins une performance de levure obtenue à partir de cette mélasse. Enfin, sur la base du modèle de prédiction entrainé, l'interface de sortie peut aussi fournir le ou les nutriments à utiliser et suivant quelles proportions pour corriger une qualité de mélasse déterminée par le modèle de prédiction entrainé.

Pour faciliter l'interaction avec l'ordinateur 601, un écran 611 et un clavier 612 peuvent être prévus et connectés au circuit informatique 603.

Les expressions telles que "comprendre", "inclure", "incorporer", "contenir", "être" et "avoir" doivent être interprétées de manière non exclusive lors de l'interprétation de la description et de ses revendications associées.

Le procédé ne se limite pas aux exemples de modes de réalisation décrits ci-avant, seulement à titre d'exemple, mais elle englobe toutes les variantes que pourra envisager l'homme de l'art dans le cadre des revendications ci-après.

Bien que décrits à travers un certain nombre d'exemples de réalisation détaillés, le procédé proposé et le dispositif pour la mise en œuvre d'un mode de réalisation du procédé comprennent différentes variantes, modifications et perfectionnements qui apparaîtront de façon évidente à l'homme de l'art, étant entendu que ces différentes variantes, modifications et perfectionnements font partie de la portée de la présente divulgation, telle que définie par les revendications qui suivent. En outre, il se peut que certains systèmes et équipements décrits ci-dessus n'incorporent pas la totalité des modules et fonctions décrits pour les modes de réalisation préférés.

## Revendications

1. Procédé de qualification de mélasse à partir d'une mesure optique, le procédé comprenant :
- à partir de spectres optiques de référence d'échantillons de mélasses distinctes, associées à des qualités de mélasse respectives, connues, construire par apprentissage automatique un modèle statistique de qualités de mélasse en fonction d'au moins une caractéristique spectrale des spectres de référence, et
- pour un spectre optique courant d'un échantillon d'une mélasse courante, à partir du modèle statistique, identifier ladite au moins une caractéristique spectrale du spectre optique courant et déterminer une qualité de la mélasse courante, ladite qualité de mélasse courante étant relative à au moins une performance de levure obtenue lorsque ladite levure est alimentée avec ladite mélasse courante,
dans lequel chaque qualité de mélasse associée respectivement à un échantillon de mélasse distincte est relative à au moins une performance de levure de référence obtenue lorsque ladite levure de référence est alimentée avec ledit échantillon de mélasse distincte.

2. Procédé selon la revendication 1, dans lequel ladite au moins une performance est basée sur au moins un critère choisi parmi :
- une capacité de multiplication de la levure alimentée par ladite mélasse courante,
- une capacité de levée de pain,
- une capacité à se conserver,
- une capacité à être séchée,
- une capacité à fixer des éléments structurels,
- une capacité à assimiler les nutriments,
- une capacité à s'adapter à des stress.

3. Procédé selon l'une quelconque des revendications précédentes, dans lequel la qualité de la mélasse courante déterminée par ledit modèle statistique est définie par au moins un score de qualité de mélasse, ledit au moins un score de qualité de mélasse étant relatif à ladite au moins une performance.

4. Procédé selon l'une quelconque des revendications précédentes, comprenant :
- illuminer un échantillon de mélasse par un premier signal lumineux émis interagissant avec l'échantillon, et collecter un deuxième signal lumineux , résultant de l'interaction entre le premier signal lumineux et l'échantillon courant ; et
- mesurer le deuxième signal lumineux, dans des bandes de fréquences choisies, pour établir un spectre optique représentatif d'une signature chimique de l'échantillon courant de mélasse.

5. Procédé selon l'une des revendications précédentes, dans lequel le spectre optique comprend un ensemble de nombre d'ondes, un ou plusieurs nombres d'ondes de l'ensemble de nombres de d'onde étant représentatifs d'un composé chimique de la mélasse.

6. Procédé selon la revendication 5, dans lequel la mélasse comporte des composés parmi au moins un sucre, un minéral, une vitamine, et le spectre optique comporte une pluralité de nombres d'onde, et dans lequel au moins un nombre d'onde caractérise la présence d'un desdits composés.

7. Procédé selon l'une des revendications précédentes, dans lequel ladite au moins une caractéristique spectrale est déterminée selon au moins un critère choisi parmi une présence ou une absence d'un nombre d'onde prédéfini, une position d'un nombre prédéfini, une valeur d'intensité d'un nombre d'onde prédéfini, un dépassement de seuil d'une amplitude d'un nombre d'onde prédéfini, une valeur maximale ou minimale d'intensité d'un nombre d'onde prédéfini, un écart d'intensité entre deux nombres d'onde prédéfini, une intensité d'un nombre d'onde prédéfini comprise entre deux valeurs d'intensité prédéterminées, ou encore des combinaisons simples ou complexes des caractéristiques spectrales précitées.

8. Procédé selon l'une des revendications précédentes, dans lequel la mesure optique est réalisée par au moins une technique parmi une spectroscopie RAMAN, une spectroscopie IR, ou une spectroscopie THZ.

9. Procédé selon l'une quelconque des revendications précédentes, dans lequel la plage de valeurs des spectres optiques de références est comprise entre 1 cm⁻¹ et 2500 cm⁻¹.

10. Procédé selon la revendication 8, dans lequel la plage de valeurs des spectres optiques de références est comprise entre 436 cm⁻¹ et 1700 cm⁻¹.

11. Procédé selon l'une des revendications précédentes, dans lequel la mélasse est une mélasse de canne ou une mélasse de betterave ou un mélange des deux, ou un mélange de produits sucriers tels qu'une mélasse et un sirop de sucre, ou un mélange de produits dont un des composants est la mélasse.

12. Programme d'ordinateur comprenant des instructions de code de programme pour l'exécution du procédé selon l'une quelconque des revendications 1 à 11 lorsque ledit programme est exécuté sur un ordinateur.

13. Support d'enregistrement non transitoire lisible par un ordinateur sur lequel est enregistré un programme pour la mise en œuvre du procédé selon l'une des revendications 1 à 11 lorsque ce programme est exécuté par un processeur.

14. Procédé de production de levures par fermentation comprenant :
- déterminer une qualité de mélasse respective pour chaque mélasse d'une pluralité de mélasses fournies en entrée de la réaction de fermentation selon le procédé de qualification de l'une quelconque des revendications 1-11, et
- alimenter la réaction de fermentation avec chaque mélasse de la pluralité de mélasses dont la qualité de mélasse respective est supérieure à une qualité prédéterminée, et pour toute ou partie des mélasses présentant une qualité de mélasse inférieure à la qualité prédéterminée, alimenter la réaction de fermentation après modification de la mélasse, ou/et rejeter la mélasse.

15. Procédé selon la revendication 14 dans lequel ladite modification de la mélasse comprend au moins une modification choisie parmi:
- mélanger la mélasse présentant une qualité de mélasse inférieure à la qualité prédéterminée, avec une autre mélasse présentant une qualité de mélasse distincte, et de sorte que le mélange de mélasses présente une qualité de mélasse supérieure à la qualité prédéterminée,
- ajouter un ou plusieurs adjuvants de sorte que la mélasse améliorée par les adjuvants présente une qualité de mélasse supérieure à la qualité prédéterminée.

## Patentansprüche

1. Verfahren zur Qualifizierung von Melasse für Produktion von Hefe ausgehend von einer optischen Messung, wobei das Verfahren umfasst:
- ausgehend von optischen Referenzspektren von Proben verschiedener Melassen, welche mit jeweiligen bekannten Melassequalitäten assoziiert sind, Erstellen, mittels maschinellen Lernens, eines statistischen Modells von Melassequalitäten in Abhängigkeit von wenigstens einem spektralen Merkmal der Referenzspektren, und
- für ein vorliegendes optisches Spektrum einer Probe einer vorliegenden Melasse, ausgehend von dem statistischen Modell, Identifizieren des wenigstens einen spektralen Merkmals des vorliegenden optischen Spektrums und Bestimmen einer Qualität der vorliegenden Melasse, wobei die Qualität der vorliegenden Melasse sich auf wenigstens eine Hefeleistung bezieht, welche erhalten wird, wenn die Hefe mit der vorliegenden Melasse versorgt wird,
wobei jede Melassequalität, welche jeweils mit einer Probe von verschiedener Melasse assoziiert ist, sich auf mindestens eine Referenz-Hefeleistung bezieht, welche erhalten wird, wenn die Referenzhefe mit der Probe von verschiedener Melasse versorgt wird.

2. Verfahren nach Anspruch 1, wobei die wenigstens eine Leistung auf wenigstens einem Kriterium basiert, welches ausgewählt ist aus:
- einer Vermehrungsfähigkeit der mit der vorliegenden Melasse versorgten Hefe,
- einer Brot-Triebfähigkeit,
- einer Fähigkeit, haltbar zu sein,
- einer Fähigkeit, getrocknet zu werden,
- einer Fähigkeit, Strukturelemente zu fixieren,
- einer Fähigkeit, Nährstoffe zu assimilieren,
- einer Fähigkeit, sich an Stress anzupassen.

3. Verfahren nach einem der vorhergehenden Ansprüche, wobei die Qualität der vorliegenden Melasse, welche durch das statistische Modell bestimmt wird, durch wenigstens einen Melassequalitätswert definiert wird, wobei sich der wenigstens eine Melassequalitätswert auf die wenigstens eine Leistung bezieht.

4. Verfahren nach einem der vorhergehenden Ansprüche, umfassend:
- Bestrahlen einer Melasseprobe mit einem ersten emittierten Lichtsignal, welches mit der Probe wechselwirkt, und Sammeln eines zweiten Lichtsignals, welches aus der Wechselwirkung zwischen dem ersten Lichtsignal und der vorliegenden Probe resultiert; und
- Messen des zweiten Lichtsignals in ausgewählten Frequenzbändern, um ein optisches Spektrum zu erstellen, welches für eine chemische Signatur der vorliegenden Melasseprobe repräsentativ ist.

5. Verfahren nach einem der vorhergehenden Ansprüche, wobei das optische Spektrum einen Satz von Wellenzahlen umfasst, wobei eine oder mehrere Wellenzahlen des Satzes von Wellenzahlen repräsentativ für eine chemische Verbindung der Melasse sind.

6. Verfahren nach Anspruch 5, wobei die Melasse Verbindungen aus wenigstens einem Zucker, einem Mineral, einem Vitamin umfasst, und das optische Spektrum eine Mehrzahl von Wellenzahlen umfasst, und wobei wenigstens eine Wellenzahl die Anwesenheit einer der genannten Verbindungen charakterisiert.

7. Verfahren nach einem der vorhergehenden Ansprüche, wobei das wenigstens eine spektrale Merkmal nach wenigstens einem Kriterium bestimmt wird, welches ausgewählt ist aus einer Anwesenheit oder einer Abwesenheit einer vordefinierten Wellenzahl, einer Position einer vordefinierten Wellenzahl, einem Intensitätswert einer vordefinierten Wellenzahl, einer Schwellenwertüberschreitung einer Amplitude einer vordefinierten Wellenzahl, einem maximalen oder minimalen Intensitätswert einer vordefinierten Wellenzahl, einer Intensitätsdifferenz zwischen zwei vordefinierten Wellenzahlen, einer Intensität einer vordefinierten Wellenzahl, welche zwischen zwei vorbestimmten Intensitätswerten umfasst ist, oder auch einfachen oder komplexen Kombinationen der vorgenannten spektralen Merkmale.

8. Verfahren nach einem der vorhergehenden Ansprüche, wobei die optische Messung durch wenigstens eine Technik aus einer RAMAN-Spektroskopie, einer IR-Spektroskopie oder einer THZ-Spektroskopie durchgeführt wird.

9. Verfahren nach einem der vorhergehenden Ansprüche, wobei der Wertebereich der optischen Referenzspektren zwischen 1 cm⁻¹ und 2500 cm⁻¹ umfasst ist.

10. Verfahren nach Anspruch 8, wobei der Wertebereich der optischen Referenzspektren zwischen 436 cm⁻¹ und 1700 cm⁻¹ umfasst ist.

11. Verfahren nach einem der vorhergehenden Ansprüche, wobei die Melasse eine Zuckerrohrmelasse oder eine Rübenmelasse oder eine Mischung der beiden ist, oder eine Mischung aus Zuckererzeugnissen wie einer Melasse und einem Zuckersirup, oder eine Mischung aus Erzeugnissen, von denen eine Komponente die Melasse ist.

12. Computerprogramm umfassend Programmcodeanweisungen zur Ausführung des Verfahrens nach einem der Ansprüche 1 bis 11, wenn das Programm auf einem Computer ausgeführt wird.

13. Nichtflüchtiges, computerlesbares Speichermedium, auf welchem ein Programm zur Durchführung des Verfahrens nach einem der Ansprüche 1 bis 11 gespeichert ist, wenn das Programm von einem Prozessor ausgeführt wird.

14. Verfahren zur Produktion von Hefe durch Fermentation, umfassend:
- Bestimmen einer jeweiligen Melassequalität für jede Melasse aus einer Mehrzahl von Melassen, welche für die Fermentationsreaktion eingangs bereitgestellt werden, nach dem Verfahren zur Qualifizierung nach einem der Ansprüche 1-11, und
- Versorgen der Fermentationsreaktion mit jeder Melasse aus der Mehrzahl von Melassen, deren jeweilige Melassequalität höher ist als eine vorbestimmte Qualität, und für alle oder einen Teil der Melassen, deren Melassequalität geringer als die vorbestimmte Qualität ist, Versorgen der Fermentationsreaktion nach Modifizierung der Melasse und/oder Verwerfen der Melasse.

15. Verfahren nach Anspruch 14, wobei die Modifizierung der Melasse wenigstens eine Modifizierung umfasst, welche ausgewählt ist aus:
- Mischen der Melasse, welche eine Melassequalität aufweist, welche geringer ist als die vorbestimmte Qualität, mit einer anderen Melasse, welche eine verschiedene Melassequalität aufweist, und derart, dass die Mischung von Melassen eine Qualität aufweist, welche höher ist als die vorbestimmte Qualität,
- Zugeben eines oder mehrerer Hilfsstoffe, derart, dass die durch die Hilfsstoffe verbesserte Melasse eine Melassequalität aufweist, welche höher ist als die vorbestimmte Qualität.

## Claims

1. A method for qualifying molasses for yeast production based on an optical measurement, the method comprising:
- based on reference optical spectra of samples of distinct molasses, associated with respective known molasses qualities, building by machine learning a statistical model of molasses qualities as a function of at least one spectral characteristic of the reference spectra, and
- for a current optical spectrum of a sample of a current molasses, based on the statistical model, identifying said at least one spectral characteristic of the current optical spectrum and determining a quality of the current molasses, said current molasses quality relating to at least one yeast performance obtained when said yeast is fed with said current molasses,
wherein each molasses quality associated respectively with a distinct molasses sample relates to at least one yeast performance obtained when said yeast is fed with said distinct molasses sample.

2. The method according to claim 1, wherein said at least one performance is based on at least one criterion selected from among:
- a multiplication capacity of the yeast fed with said current molasses,
- a bread leavening capacity,
- a capacity to be preserved,
- a capacity to be dried,
- a capacity to fix structural elements,
- a capacity to assimilate nutrients,
- a capacity to adapt to stresses.

3. The method according to any one of the preceding claims, wherein the quality of the current molasses determined by said statistical model is defined by at least one molasses quality score, said at least one molasses quality score relating to said at least one performance.

4. The method according to any one of the preceding claims, comprising:
- illuminating a molasses sample with a first emitted light signal interacting with the sample, and collecting a second light signal, resulting from the interaction between the first light signal and the current sample; and
- measuring the second light signal, in selected frequency bands, to establish an optical spectrum representative of a chemical signature of the current molasses sample.

5. The method according to one of the preceding claims, wherein the optical spectrum comprises a set of wavenumbers, one or more wavenumber(s) of the set of wavenumbers being representative of a chemical compound of the molasses.

6. The method according to claim 5, wherein the molasses includes compounds at least one from among a sugar, a mineral, a vitamin, and the optical spectrum includes a plurality of wavenumbers, and wherein at least one wavenumber characterises the presence of one of said compounds.

7. The method according to one of the preceding claims, wherein said at least one spectral characteristic is determined according to at least one criterion selected from among a presence or an absence of a predefined wavenumber, a position of a predefined number, an intensity value of a predefined wavenumber, overpassing of a threshold of an amplitude of a predefined wavenumber, a maximum or minimum intensity value of a predefined wavenumber, an intensity deviation between two predefined wavenumbers, an intensity of a predefined wavenumber comprised between two predetermined intensity values, or simple or complex combinations of the aforementioned spectral characteristics.

8. The method according to one of the preceding claims, wherein the optical measurement is carried out by at least one technique from among a RAMAN spectroscopy, an IR spectroscopy, or a THZ spectroscopy.

9. The method according to one of the preceding claims, wherein the range of values of the reference optical spectra is comprised between 1 cm⁻¹ and 2,500 cm⁻¹.

10. The method according to claim 8, wherein the range of values of the reference optical spectra is comprised between 436 cm⁻¹ and 1,700 cm⁻¹.

11. The method according to one of the preceding claims, wherein the molasses is a cane molasses or a beet molasses or a mixture of both, or a mixture of sugar products such as a molasses and a sugar syrup, or a mixture of products one of the components of which is the molasses.

12. A computer program comprising program code instructions for executing the method according to any one of claims 1 to 11 when said program is executed on a computer.

13. A non-transitory computer-readable recording medium on which a program is recorded for implementing the method according to one of claims 1 to 11 when this program is executed by a processor.

14. A method for producing yeasts by fermentation comprising:
- determining a respective molasses quality for each molasses of a plurality of molasses supplied at the input of the fermentation reaction according to the qualification method of any one of claims 1-11, and
- feeding the fermentation reaction with each molasse of the plurality of molasses whose respective molasses quality is higher than a predetermined quality, and for all or part of the molasses having a molasses quality lower than the predetermined quality, feeding the fermentation reaction after modification of the molasses, or/and discarding the molasses.

15. The method according to claim 14 wherein said modification of the molasses comprises at least one modification selected from among:
- mixing the molasses having a molasses quality lower than the predetermined quality, with another molasses having a distinct molasses quality, and so that the molasses mixture has a molasses quality higher than the predetermined quality, and
- adding one or more additive(s) so that the molasses improved by the additives has a molasses quality higher than the predetermined quality.
